# EUROPEAN PATENT APPLICATION

(11) **EP 4 289 546 A1**
(43) Date of publication of application: **13.12.2023**
(21) Application number: 22020264.2
(22) Date of filing: 08.06.2022
(51) Int. Cl.: B23K 31/02, B23K 26/14, B23K 10/00, B23K 9/32, B23K 26/12, G01N 33/00, B23K 9/16

(54) **AUTOMATED ONLINE MONITORING OF GAS QUALITY**

(71) Applicant: Linde GmbH, 82049 Pullach (DE)
(72) Inventor: Hussary, Nakhleh A., 82049 Pullach (DE); Bern, Robert, 82049 Pullach (DE)
(74) Representative: Lu, Jing

(57) **Abstract**

The present invention relates to a system (1)for automated analysis of a gas (G) at full operating gas volume during a joining or cutting process, comprising: a gas analyzing unit (4), a torch (2) for conducting the joining or cutting process, the torch (2) comprising a gas nozzle (3) being configured to discharge said gas, the torch (2) being further configured to discharge the gas (G) during said joining or cutting process at said operating gas volume flow, wherein the torch (2) comprises a conduit (46) to extract at least a partial stream of said gas (G) from the gas nozzle (3), said conduit being configured to be connected to the gas analyzing unit (4) to provide a flow connection between the torch (2) and the gas analyzing unit (4) to allow passing of said extracted gas (G) to the gas analyzing unit (4).

## Description

The present invention relates to a system and a method for automated monitoring of gas quality, particularly for a joining or cutting process. Such a joining process can be a welding or brazing process, particularly gas metal arc welding (GMAW), tungsten inert gas welding (TIG), plasma welding, laser cladding, laser welding, plasma brazing, arc brazing, plasma spraying. The cutting process can be a plasma cutting process.

Gas analysis is frequently used in order to make sure that gas compositions used in a process are up to the desired specification. Corresponding gas analyzing devices can comprise the capability of remote data transmission, data recording, alerting, and remote access.

Up to now, only random samples have been taken at the gas nozzles used in welding processes during troubleshooting. However, an automated and standardized measuring method is missing.

Particularly, when welding or brazing using e.g. the above-stated procedures, welding defects can occur due to contamination of the welding gases. Even if the delivery condition of the gases is proper, the downstream components such as pressure regulators, gas lines, hose lines, wire liners, torch cooling, gaskets, fittings, connections, gas mixers, can lead to contamination of the gases.

In the frame work of the present invention, the respective gas can e.g. be provided from bottles or bundles or from liquid tanks that are used either directly to a process or are used for mixing and storing in buffer tanks. Particularly, gas lines in form of ring lines can be used in a facility that distributes the gas in the various parts of a manufacturing building. Instead of a ring line also a gas line in form of a single direction line to an end point with multiple users on the line can be used. A joining (e.g. welding) or cutting process is then supplied from the ring line or single direction line by individual gas hoses.

Often, there is no qualification of the used gas composition before processing (e.g. welding, brazing). This is more critical after a period of interrupted work such as overnight, over the weekend or a long production break, e.g., due to holidays.

Possible causes for welding or brazing defects are typically moisture, oxygen and nitrogen that can escape through diffusion-open gas hoses or seals and can get into the gas line system after longer periods of downtime.

Furthermore, leaks in the torch cooling system can cause welding defects. Depending on the exit velocity and given gas flows, gas can be discharged or air can be sucked in along the welding wire liner. Further, improper hose connections and leaks may generally lead to welding defects, as well as defective gas mixers or incorrectly set pressures.

In the EN ISO 14175 standard, the gas purities and mixing accuracies are described as delivered, but not at the time of consumption or the point of use. The time of consumption is when the gas flows through the gas nozzle of the respective welding torch / apparatus, affecting the welding process. The point of use refers to the critical location of the gas shielding of the arc from the atmosphere (e.g. the process zone).

Based on the above, it is an object of the present invention to provide an improved system and method for automated analysis and monitoring of a gas used in a joining or cutting process.

This problem is solved by a system having the features of claim 1 and a method having the features of claim 8. Preferred embodiments of these aspects of the present invention are stated in the sub claims and are described below.

According to claim 1, a system is disclosed for automated analysis and monitoring of a gas, preferably at full operating gas volume flow as used during a joining or cutting process, or at a fraction of said full operating gas volume flow, the system comprising:
- a gas analyzing unit,
- a torch for conducting the joining or cutting process, the torch comprising a gas nozzle being configured to discharge said gas, the torch being further configured to discharge the gas during said joining or cutting process at said full operating gas volume flow or at said fraction of the full operating gas volume flow,
- wherein the torch comprises a conduit to extract at least a partial stream of said gas from the gas nozzle, said conduit being configured to be connected to the gas analyzing unit to provide a flow connection between the torch and the analyzing unit to allow passing of said extracted gas to the gas analyzing unit, preferably when the gas is discharged at said full operating gas volume flow, and
- wherein the gas analyzing unit is configured to analyze the gas with respect to at least one impurity and/or at least one concentration of a component of the gas.

Thus, the present invention allows to continuously analyze the respective gas (particularly before starting the process, during the process, and after completion of the process).

According to an embodiment, the gas is discharged by the gas nozzle on the workpiece(s) to be joined (e.g. welded) or cut to protect the weld pool/molten material from oxidation.

In some processes multiple gases and corresponding lines may be used, namely in particular plasma gas, shielding gas, and in some processes also a further gas such as a focus gas. Particularly, Plasma Arc Welding (PAW) and plasma cutting processes use a plasma gas and a shield gas. In some implementations of PAW, a focusing gas may be also be used. GMAW and GTAW (also known as TIG) typically use a shielding gas. In some implementations of TIG a focusing gas may also be used.

The gas analyzing unit may analyze all gas types that are being used (e.g. plasma gas, shielding gas, focusing gas). Particularly, the analysis may happen simultaneously (by having multiple sensors) or successively (e.g. one sensor that measures all gas lines one after the other). The system according to the present invention can also be used to measure the flow of orbital welding (i.e. a special welding system for pipes) or closed welding systems (enclosed box or housing).

According to a preferred embodiment however, for GMAW (gas metal arc welding) or GTAW (gas tungsten arc welding), which covers 90+% of all welding cases, the shielding gas is the main gas component to be analyzed/verified.

Particularly, the joining process can be one of: a welding process, a brazing process, GMAW, TIG, plasma welding, laser welding, laser cladding, plasma spraying, plasma brazing, arc brazing. Further, the cutting process can be plasma cutting.

According to yet another preferred embodiment of the system, the gas nozzle comprises an opening through which said gas is released into the ambient atmosphere, wherein the conduit comprises an opening for sucking said partial stream into the conduit, wherein the opening of the gas nozzle and the opening of the conduit comprise a distance from one another in the range from 1 cm to 3cm.

Due to said distance, the risk of damaging the conduit by heat from the arc or the process or of causing any disturbance to the symmetry of the flow in the nozzle is significantly reduced.

Furthermore, according to a preferred embodiment of the system according to the present invention, the conduit is arranged to extract said partial stream of the gas from a middle region in the torch.

Furthermore, according to a preferred embodiment of the system, the conduit is arranged to extract said partial stream of the gas from the back end of the torch opposite said gas nozzle.

According to yet another preferred embodiment, the system comprises a torch lead connected to the torch for passing said gas into the torch. Apart from a torch lead for gas, the system can comprise torch leads for carrying current, cooling water, and wire.

According to a preferred embodiment, the conduit is arranged to extract said partial stream of the gas from a middle location in said torch lead, the middle location being arranged between a first connection of the torch lead to a housing of the system, the housing accommodating a power supply and a main gas control, and a second connection of the torch lead to the back end of the torch.

According to a further preferred embodiment, the conduit is arranged to extract said partial stream of the gas from a location in the torch lead adjacent the first connection.

Furthermore, according to a preferred embodiment, multiple conduits may be used to extract a partial stream of the gas, respectively, from different flow paths (e.g. plasma gas, shielding gas, focusing gas etc.).

According to a further preferred embodiment of the present invention, said partial stream of the gas comprises volume flow in the range from 0.25 I/min to 5 I/min.

Furthermore, according to a preferred embodiment, said partial stream is at least 1/30, particularly at least 1/20, particularly at least 1/10, particularly at least 1/5 of the full operating gas volume flow.

Furthermore, preferably, an inner diameter of the conduit is minimized to increase the measurement reaction time of the system. Further, preferably the total volume of the interior of the conduit is minimized, too. Furthermore, the distance from the end of the conduit to the gas analyzing unit is minimized to decrease the reaction time of the sensor.

Further, particularly, the impurities considered for welding defects, which depend on the material, can be chemical agents such as H₂O, O₂, N₂, CO₂. Furthermore, concentrations of e.g. CO₂, O₂, He, H₂, N₂ in the gas can be too low or too high. There is the option to extrapolate N₂ over the detected O₂ content, if O₂ is not a basic component of a gas being used. These possible impurities and also the mixing accuracy (e.g. concentration of the respective component) are preferably measured with a real-time gas analyzing unit in the vicinity of the gas nozzle of the torch. If atmospheric gas is leaking into the system and an oxygen contamination is measured, one may be able to estimate the contamination by N₂ by applying the ratio of oxygen and to N₂ in air, i.e., 3.7 times more N₂ contamination than oxygen contamination.

According to an embodiment, the system is configured not to start the joining process (particularly welding or brazing process) or cutting process until the respective impurity is below a predefined limiting value. Furthermore, particularly, joining (welding or brazing) processes as well as cutting processes are also only started if the gas has its component's concentrations within the specified range.

According to a preferred embodiment of the system, the system comprises a control unit operatively connected to the analyzing unit, wherein the control unit is configured to initiate the joining or cutting process in case said at least one impurity is below a predefined limiting value and/or said concentration is within a desired range, and/or wherein the control unit is configured to initiate a purging of the gas nozzle in case said at least one impurity is above a predefined limiting value and/or said concentration is outside a desired range.

According to a preferred embodiment, the gas analyzing unit is configured to analyze the gas with the actual gas volume flow as it is used in the welding or brazing process. This gas volume flow is denoted as full operating gas volume flow.

According to a preferred embodiment of the invention, the gas analyzing unit is configured to analyze the gas with the full operating gas volume flow as it is used in a joining or cutting process, particularly to ensure any leaks are properly scaled to the actual flow rate being used in the process. No special extraction of a low flow from the torch to the gas analyzing unit is required, as currently available gas analyzing units can only handle a small amount of gas flow and special extraction is required. Particularly, a small leak in a low flow process has a larger impact compared to a high flow process. Therefore, in the context of the present invention, the analysis is preferably done at full operating gas volume flow of the gas as being used during an actual joining or cutting process, i.e., the full operating gas volume flow is fed to the gas analysing unit via said connection element, so that the gas analyzing unit can properly analyze the contents. Feeding the full intended operating gas volume flow also has the benefit of allowing to capture any leaks or impurities that make it into the system (through any of the sub-components throughout the joining or cutting process) more easily. Internally, the gas analyzing device can extract a fraction of the gas volume flow needed for the respective sensor. However, the present invention also applies to situations where not the full operating gas volume flow is used, but a percentage of the full operating gas volume flow, particularly at least 10%, particularly at least 20%, particularly at least 30%, particularly at least 40%, particularly at least 50%, particularly at least 60%, particularly at least 70%, particularly at least 80 %, particularly at least 90%.

According to an embodiment of the system, the processing unit comprises a robotic or mechanized arm configured to move the torch to working site where a joining or cutting procedure shall be performed, Since the analyzing unit is in flow connection via said conduit with the gas nozzle, the gas can be analyzed at every working site the torch is positioned.

Furthermore, in an embodiment, the system is configured to purge the torch by discharging gas through the gas nozzle for a predefined amount of time in case the processing unit has been idle for more than a predefined idle time.

Particularly, extracting gas from the front of the torch head using the torch-integrated conduit ensures that the gas composition is sampled and measured at the most critical location. The sampling can be achieved through embedding an extraction apparatus on the front of the torch, such as said conduit, to ensure that sampling is being done from the proper end location and that the gas being extracted is properly sealed and then routed to the gas analyzing unit. Particularly, the conduit is embedded in the torch and extends from the front of the troch back to the gas analyzing unit. Therefore, the critical location requiring the proper gas composition (or requiring lack of contamination) is in flow communication with the gas analyzing unit.

According to a further embodiment, the system comprises a power source electrically connectable to the torch for allowing the torch to perform the joining or cutting process (e.g. by generating an arc), wherein the gas analyzing unit is integrated together with said power source into a common housing of the system. Integrating the gas analysing unit and power source in this fashion ensures the reduction of needed infrastructures for powering the sensors, extracting the data, processing the data and decision making (starting or stopping the process) as well as porting/loading the data into a Quality Management System (QMS) with time stamping for traceability.

According to yet another aspect, a method is disclosed for automated analysis and monitoring of a gas at full operating gas volume flow as used during a joining or cutting process, or at a fraction of said full operating gas volume flow, the method comprising the steps of:
- providing a gas analyzing unit,
- providing a torch for conducting the joining or cutting process, the torch comprising a gas nozzle being configured to discharge said gas, the torch being further configured to discharge the gas during said joining or cutting process at said full operating gas volume flow or at said fraction of the full operating gas volume flow,
- extracting at least a partial stream of said gas from the gas nozzle via a conduit integrated into the torch when the gas is discharged at said full operating gas volume flow and passing of said gas to the gas analyzing unit, and
- analyzing the gas with the gas analyzing unit with respect to at least one impurity contained in the gas and/or at least one concentration of a component of the gas.

According to a preferred embodiment of the invention, the gas analyzing unit is configured to analyze the gas with the actual gas volume flow as it is used in a joining or cutting process, particularly to ensure any leaks are properly scaled to the actual flow rate being used in the process. This gas volume flow is denoted as full operating gas volume flow. However, the present invention also applies to situations where not the full operating gas volume flow is used, but a percentage of the full operating gas volume flow, particularly at least 10%, particularly at least 20%, particularly at least 30%, particularly at least 40%, particularly at least 50%, particularly at least 60%, particularly at least 70%, particularly at least 80 %, particularly at least 90%.

However, using the full operating gas flow has the benefit of ensuring that the actual concentration of the contaminants or the concentrations can be easily determined when running the respective joining or cutting process. Using the full operating gas flow allows one to omit calculations necessary when using percentages of the full operating gas volume flow. Furthermore, the full operating gas volume flow will ensure that leaks are accounted for correctly.

According to a preferred embodiment of the method, the method further comprises providing a control unit operatively connected to the analyzing unit, wherein the control unit initiates the joining or cutting process in case said at least one impurity is below a predefined limiting value and/or said concentration is within a desired range, and/or wherein the control unit initiates a purging of the gas nozzle in case said at least one impurity is above a predefined limiting value and/or said concentration is outside a desired range.

According to an embodiment of the method, wherein the system is configured to purge the torch by discharging said gas through the gas nozzle for a predefined amount of time in case the processing unit has been idle for more than a predefined idle time.

Further, in an embodiment of the method the joining process is one of: a welding process, a brazing process, GMAW, TIG, plasma welding, plasma spraying. arc brazing, plasma brazing. Furthermore, according to an embodiment of the method, the cutting process is plasma cutting.

In the following, embodiments of the present invention as well as further features and advantages shall be explained with reference to the Figures, wherein
- Fig. 1: shows a schematic illustration of an embodiment of the system and method according to the present invention;
- Fig. 2: shows a block diagram of an embodiment of the method according to the present invention;
- Fig. 3: shows further block diagrams relating to purging of the torch according to embodiments of the method according to the present invention;
- Fig. 4: shows an embodiment of the torch with integrated conduit to extract gas for analysis upstream the gas nozzle from within the torch; and
- Fig. 5: shows an embodiment of the system having a gas analyzing unit integrated into a power supply of the system.

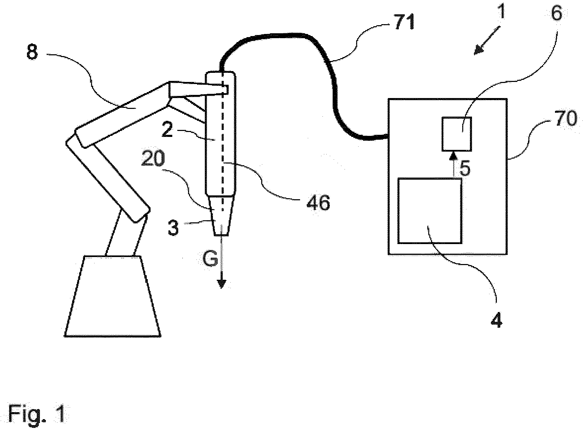

Fig. 1 shows a schematic illustration of a system 1 for automated analysis and monitoring of a gas G used in a joining process (such as a welding or brazing process) or in a cutting process (e.g. plasma cutting) at full operating gas volume flow, wherein the system 1 comprises a gas analyzing unit 4, a control unit 6, and a torch 2 for conducting the joining or cutting process, the torch 2 comprising a gas nozzle 3 being configured to discharge said gas G, the torch 2 being further configured to discharge the gas G during the joining or cutting process at said operating gas volume flow, wherein the torch 2 is connected to the gas analyzing unit 4 to provide a flow connection between the torch 2 and the gas analyzing unit 4 to allow passing of said gas G to the gas analyzing unit 4, wherein the gas analyzing unit 4 is configured to analyze the gas G with respect to at least one impurity and/or at least one concentration of a component of the gas G. Particularly, the gas analyzing unit 4 can be configured to generate an output signal 5 and to transmit the output signal 5 to the control unit 6 allowing the processing unit 6 to conduct the joining or cutting process in case said at least one impurity is below a predefined limiting value and/or said concentration is within a desired range.

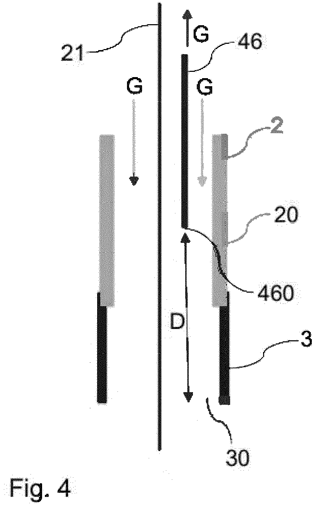

Particularly, as indicated in Fig. 1 and the detail shown in Fig. 4, the present invention allows automated measurement and monitoring (qualification) of the gas purity and also the mixing accuracy directly at the gas nozzle 3 for joining and cutting processes, particularly welding processes such as GMAW or arc brazing process at full operating gas volume flow of the torch 2 (also other processes can be used, see above). As shown in Fig. 4 the torch 2 is preferably adapted such that the gas G can be extracted from the front of the torch head 20 to ensure that the gas composition is sampled and measured at the most critical location. Particularly, the gas nozzle 3 comprises an opening 30 through which said gas G is released into the ambient atmosphere/on the workpiece to be processed, wherein extraction of a partial gas stream to be analyzed is conducted via a conduit 46 integrated into the torch 2, which conduit comprises an opening 460 for sucking said partial stream into the conduit 46 in the vicinity of the gas nozzle 3. Particularly, a distance D between the opening 30 of the gas nozzle 3 and an opening 460 of the conduit 46 is in the range from 0 cm to 3cm, particularly 1 cm to 3 cm. According to the invention, the extraction apparatus, e.g. conduit 46, is embedded as part of the fundamental design of the torch 2. These torches 2 may be torches 2 that are configured for conducting the joining and cutting processes mentioned herein. The extraction apparatus, e.g. conduit 46, can also be embedded in an already existing torch 2 (such as a GMAW, TIG torch, etc.).

Furthermore, the torch 2 can be adapted to advance an electrode 21 (e.g. in case the joining process is gas metal arc welding or GMAW). Sampling the gas G in the vicinity of the gas nozzle 3 represents the final stage and the actual gas G being seen by the respective joining (e.g. welding or brazing) process. The sampled gas G is analyzed in the gas analyzing unit 4 to ensure the accuracy of the mix and that it is free of impurities. Particularly, impurities are defined as trace gases or molecules not intended to be part of the gas G, for example this may be gases such as oxygen, nitrogen, CO₂ or may be humidity or other molecules (see also above). The sampling is achieved through embedding an extraction apparatus on the front of the torch 2, such as a conduit 46, to ensure that sampling is being done from the proper end location and that the gas G being extracted is properly sealed and then routed to the gas analyzing unit 4.

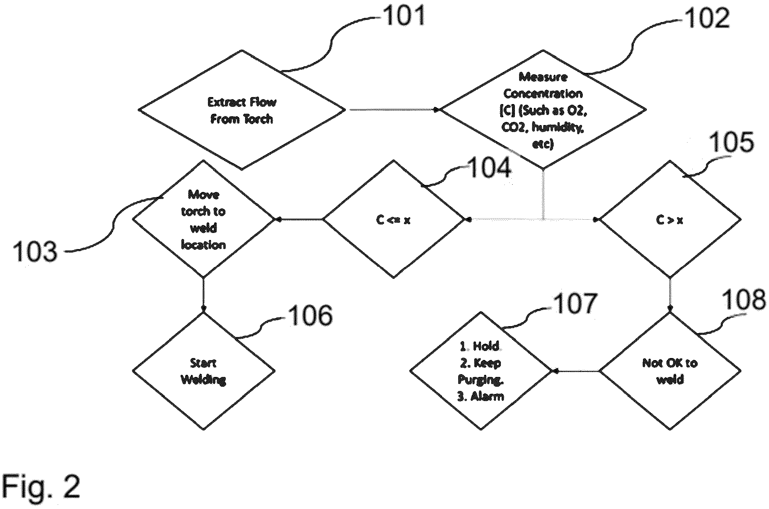

Particularly, as shown in Fig. 1, the gas analyzing unit 4 is connected to the control unit 6 so that the system 1 can start when the measurement of the analyzing unit being indicative of at least one impurity of the gas G and/or concentrations of components of the gas G shows that a pre-defined criterion is met. The control unit 6 controlling the joining process then receives an "OK-to-Proceed" signal 5 indicating to the control unit 6 that a stable composition level of gas G with the acceptable parameters can be provided to the gas nozzle 3. Fig. 2 illustrates the signal flow and process steps of an embodiment of the method according to the present invention. According thereto, conduit 46 extracts a partial stream of an operating gas volume flow of the gas nozzle 3 (101) and measures a concentration [c] such as O₂, CO₂, humidity etc (102). In case this impurity concentration c is above a certain limit x (105), the joining (here e.g. welding/brazing) is not started, but further measures can be performed to reduce impurities, such as purging. An alarm informing about the impurities in the gas G can be issued to the user (107). In case concentration c is below the threshold x (104), the torch 2 is moved to the welding/brazing location (105) and welding/brazing is started (106). This procedure can also apply to a cutting process.

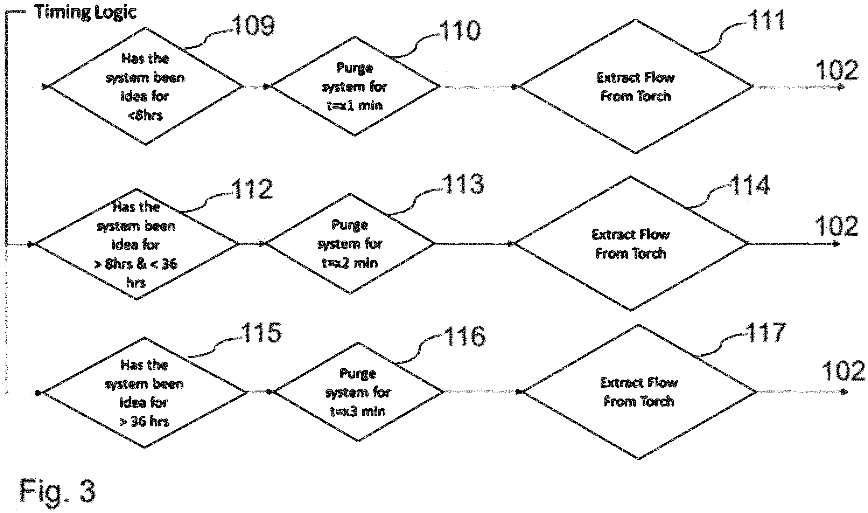

Fig. 3 shows a further embodiment of the method relating to purging of the system 1 in case the system has been idle for a certain time. For this a timing logic is implemented into the system 1 according to which in case the system has been idle for less than 8 hours (109) the system 1 is purged for at least a minute (110). Afterwards gas G is extracted from the torch 2 (111) and the method continues with step 102 shown in Fig. 1.

Furthermore, in case the system 1 has been idle for more than 8 hours but less than 36 hours (112) the system 1 is purged for at least two minutes (113). Afterwards gas G is extracted from the torch 2 (114) and the method continues with step 102 shown in Fig. 1.

Furthermore, in case the system 1 has been idle for more than 36 hours (115) the system 1 is purged for at least three minutes (116). Afterwards gas G is extracted from the torch 2 (117) and the method continues with step 102 shown in Fig. 1.

Particularly, the contaminants considered for welding (or brazing) defects, which depend on the material, are chemical agents such as H₂O, O₂, N₂, CO₂. Further components of the gas may have concentrations of e.g. CO₂, O₂, He, H₂, N₂being too low or too high in the gas mixture. Particularly, there is the option to extrapolate N₂ over the detected O₂ content, if O₂ is not a basic component of the gas (see also above). These possible contaminations and also mixing accuracy are preferably measured in real-time with the gas analyzing unit 4 directly at the gas nozzle 3. Welding/brazing processes are not released until the specifications regarding impurities are met. Further, welding/brazing processes are also only released if the specified gas mixture is within the specified tolerances.

Preferably, the gas G is at least analyzed prior to start-up after a weekend or after a pre-defined shutdown period in order to release a process. Intermediate measurements and measurements after completion of a joining (e.g. welding) process or cutting process may also be useful. Particularly, welding and brazing processes can thus be monitored and subjected to a minimum standard. At present, all decisive welding or brazing parameters can be monitored and documented, but the gas G that enters the process directly through the gas nozzle 3 is not taken into account. However, this gas contributes to the success or failure of a weld.

Possible applications of the welding or brazing processes monitored with the system or method according to the present invention can be pressure pipeline construction, automotive, tank construction, crane construction, bridge construction, formwork construction, ropeway construction etc.

Particularly, in the framework of the present invention, the extraction and analysis of the gas G can be done at one of multiple stages of the process such as before starting the process or after the processing (e.g. welding or brazing) has been completed. The extraction and analysis may also be done during the process. Remedies maybe implemented to activate purge sequence to bring lower the contamination. The purge sequence may be increased progressively.

The extraction and analysis may be continuous or may be set at given intervals. The intervals may vary and can be a function of the process itself, the production time or other factors.

The gas G extracted through the extraction apparatus 46 represents a true sample of the gas G. It will include any and all cumulative impurities introduced at all stages in the system 1 (from the supply line, to the connections to the process itself, the gas control unit, the connection of a torch lead 71 to the power source 7 and gas control unit up to the torch head 20 itself (see also Fig. 1).

The gas sample is preferably extracted when the process is flowing gas G at its recommended gas volume flow so as to properly measure the concentrations of all the components of the gas G and the impurities therein. Therefore, extracting from the critical location that is flowing too little or too high of a flow may overestimate or underestimate the concentrations and therefore provide erroneous input for the decision-making process (this being either done by the welder or by an automated system in mechanized or robotic applications). Further, preferably, all the data is analyzed and recorded with a time stamp. Therefore, the gas composition conforms to the ISO 14175 and ISO 3834.

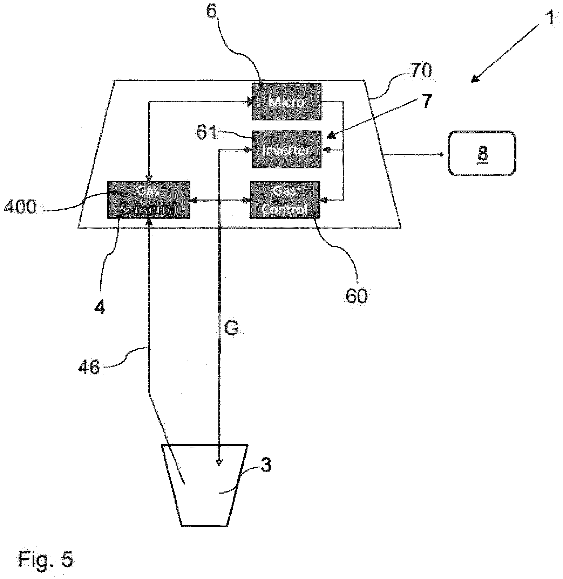

Further, Fig. 5 illustrates a further embodiment of a system 1 according to the present invention. Here, the gas analyzing unit 4, and particularly its gas sensors, are integrated into a housing 70 of the system 1 that also accommodates a welding power source 7 ensuring the reduction of needed infrastructures for powering the sensors, extracting the data, processing the data and decision making (starting or stopping the process) as well as porting/loading the data into a Quality Management System (QMS) 8 with time stamping for traceability. This significantly reduces the infrastructure cost and the cost in developing interfaces and communication protocols to communicate with the power supply and the quality management system.

Particularly, the housing 70 of the power source 7 houses the sensors 400 of the gas analyzing unit 4, wherein the power source 7 provides the needed power to energize the sensors 400 and the sensor subcomponents. The sensors 400 may be oxygen concentration sensors, CO₂ infrared sensors, humidity sensors, MEMS (micro electro mechanical systems), hydrogen sensors or others. The subcomponents of the sensor(s) 400 may include small flow meters/controllers, pressure and temperature sensors and others.

Further, the system comprises a micro-controller 6 embedded in the housing 70 of the power supply 7 to provide a control logic for running and/or coordinating not only an inverter power module 61 of the power supply 7 and a gas controller 60 for controlling gas volume flow of the gas G, but also the embedded sensors 400 of the gas analyzing unit 4 (alternatively single gas sensors).

Furthermore, within the scope of this invention is having a unitary body module that houses the gas sensors 400 embedded in it and intended to fit (mechanically and electrically) within the power source's 7 housing 70 or being attached to the power source 7 as a submodule. The attachment may be internal or external.

Within the scope of this invention is having the single sensor 400 or multiple sensors 400 installed in the housing 70 of the power source 7 natively and not being housed in their own unitary body. The gas sensors 400 or the unitary body of the gas analyzing unit 4 is in fluid communications with the torch 2 being used (i.e. welding torch, cutting torch, spraying torch, etc.). This may be provided as internal to the torch lead 71 and the torch 2 itself or the gas may be sampled externally.

The data collected from the gas sensors 400 may be saved to an on-board memory storage device or directly uploaded to a server or the cloud through ethernet LAN, WIFI, cellular network or other means.

Particularly, the data collected from the gas sensors 400 are time-stamped and synchronized with the native data collected from the process such as the voltage, current and gas volume flows as well as the wire feed speeds and other signals that may be integrated into the power source 7 (i.e. welding speed).

The data may also be ported to an external server or cloud service through the main power supply interface protocols and implemented into the manufacturing quality management system [QMS] 8 for compliance with ISO 14175, ISO 3834 and ISO 1090.

## Claims

1. A system (1) for automated analysis of a gas (G), preferably at full operating gas volume flow as used during a joining or cutting process, or at a fraction of said full operating gas flow, comprising:
- a gas analyzing unit (4),
- a torch (2) for conducting the joining or cutting process, the torch (2) comprising a gas nozzle (3) being configured to discharge said gas, the torch (2) being further configured to discharge the gas (G) during said joining or cutting process at said operating gas volume flow or at said fraction of the full operating gas volume flow,
- wherein the torch (2) comprises a conduit (46) to extract at least a partial stream of said gas (G) from the gas nozzle (3), said conduit being configured to be connected to the gas analyzing unit (4) to provide a flow connection between the torch (2) and the gas analyzing unit (4) to allow passing of said extracted gas (G) to the gas analyzing unit (4), and
- wherein the gas analyzing unit (4) is configured to analyze the gas (G) with respect to at least one impurity and/or at least one concentration of a component of the gas (G).

2. The system according to claim 1, wherein the system comprises a control unit (6) operatively connected to the analyzing unit (4), wherein the control unit (6) is configured to initiate the joining or cutting process in case said at least one impurity is below a predefined limiting value and/or said concentration is within a desired range, and/or wherein the control unit (6) is configured to initiate a purging of the gas nozzle (3) in case said at least one impurity is above a predefined limiting value and/or said concentration is outside a desired range.

3. The system according to one of the preceding claims, wherein the gas nozzle (3) comprises an opening (30) through which said gas (G) is released into the ambient atmosphere, wherein the conduit (46) comprises an opening (460) for sucking said partial stream into the conduit (46), wherein the opening (30) of the gas nozzle (3) and the opening (460) of the conduit (46) comprise a distance (D) from one another in the range from 0 cm to 3 cm, preferably 1 cm to 3 cm.

4. The system according to one of the preceding claims, wherein the system (1) is configured to purge the torch (2) by discharging said gas (G) through the gas nozzle (3) for a predefined amount of time in case the torch (2) has been idle for more than a predefined idle time.

5. The system according to one of the preceding claims, wherein the joining process is one of: a welding process, a brazing process, GMAW, TIG, plasma welding, laser welding, laser cladding, plasma spraying, arc brazing, plasma brazing; and/or wherein the cutting process is plasma cutting.

6. The system according to one of the preceding claims, wherein the system (1) comprises a power source (7) electrically connectable to the torch (2) for allowing the torch (2) to conduct the joining or cutting process [particularly to generate an arc].

7. The system according to claim 6, wherein the system comprises a housing (70) into which the gas analyzing unit (4) is integrated, wherein the power source (7) is integrated into this housing (70), too.

8. A method for automated analysis of a gas (G), preferably at full operating gas volume flow as used during a joining or cutting process, or at a fraction of said full operating gas flow, the method comprising the steps of:
- providing a gas analyzing unit (4),
- providing a torch (2) for conducting the joining or cutting process, the torch (2) comprising a gas nozzle (3) being configured to discharge said gas (G), the torch (2) being further configured to discharge the gas (G) during said joining or cutting process at said operating gas volume flow or at said fraction of the full operating gas volume flow,
- extracting at least a partial stream of said gas (G) from the gas nozzle (3) via a conduit (46) integrated into the torch (2) when the gas (G) is discharged at said full operating gas volume flow and passing of said gas (G) to the gas analyzing unit (4), and
- analyzing the gas (G) with the gas analyzing unit (4) with respect to at least one impurity and/or at least one concentration of a component of the gas (G).

9. The method according to claim 8, wherein the method further comprises providing a control unit (6) operatively connected to the analyzing unit (4), wherein the control unit (6) initiates the joining or cutting process in case said at least one impurity is below a predefined limiting value and/or said concentration is within a desired range, and/or wherein the control unit (6) initiates a purging of the gas nozzle (3) in case said at least one impurity is above a predefined limiting value and/or said concentration is outside a desired range.

10. The method according to one of the claims 8 to 9, wherein the joining process is one of: a welding process, a brazing process, GMAW, TIG, plasma welding, laser welding, laser cladding, plasma spraying, arc brazing, plasma brazing; and/or wherein the cutting process is plasma cutting.
